# EUROPÄISCHE PATENTSCHRIFT

(11) **EP 1 874 760 B1**
(45) Veröffentlichungstag und Bekanntmachung des Hinweises auf die Patenterteilung: **06.10.2010**
(21) Anmeldenummer: 06707594.5
(22) Anmeldetag: 17.03.2006
(51) Int. Cl.: C07D 401/14, C07D 403/12, C07D 403/14, C07D 407/14, A61P 35/00

(54) **(lH-IND0L-7-YL)-(PYRIMIDIN-2 -YL-AMINO) METHANON DERIVATE UND VERWANDTE VERBINDUNGEN ALS IGF-Rl INHIBITOREN ZUR BEHANDLUNG VON KREBS**
(LH-IND0L-7-YL)-( (PYRIMIDIN-2 -YL-AMINO) METHANONE DERIVATIVES AND RELATED COMPOUNDS AS IGF-RL INHIBITORS FOR TREATING CANCER
DERIVES DE (1H-INDOL-7-YL)-(PYRIMIDIN-2-YL-AMINO)METHANONE ET COMPOSES APPARENTES UTILISES COMME INHIBITEURS D'IGF-R1 POUR TRAITER LE CANCER

(30) Priorität: 12.04.2005 DE 102005016634
(43) Veröffentlichungstag der Anmeldung: 09.01.2008
(73) Patentinhaber: Merck Patent GmbH, 64293 Darmstadt (DE)
(72) Erfinder: HEINRICH, Timo, 64823 Gross-Umstadt (DE); BLAUKAT, Andree, 64367 Muehltal (DE); KORDOWICZ, Maria, 64347 Griesheim (DE)
(86) Internationale Anmeldenummer: PCT/EP2006/002470
(87) Internationale Veröffentlichungsnummer: WO 2006/108487

(56) Entgegenhaltungen:
- WO-A-03/018021
- DE-A1- 2 142 353
- DE-A1- 2 847 662
- HOLT ET AL: "Benzophenone- and Indolecarboxylic Acids: Potent Type-2 Specific Inhibitors of Human Steroid 5.alpha.-Reductase" JOURNAL OF MEDICINAL CHEMISTRY, Bd. 38, Nr. 1, 1995, Seiten 13-15, XP002384662
- CINQUE ET AL: "Structure-Activity Relationship of New Growth Inhibitors of Trypanosoma cruzi" JOURNAL OF MEDICINAL CHEMISTRY, Bd. 41, Nr. 9, 1998, Seiten 1540-1554, XP002384663
- SARMIENTO ET AL: "Structure-Based Discovery of Small Molecule Inhibitors Targeted to Protein Tyrosine Phosphatase 1B" JOURNAL OF MEDICINAL CHEMISTRY, Bd. 43, Nr. 2, 2000, Seiten 146-155, XP002384664

## Beschreibung

Die Erfindung betrifft einzelne Verbindungen gemäß Anspruch 1 von der Formel I, 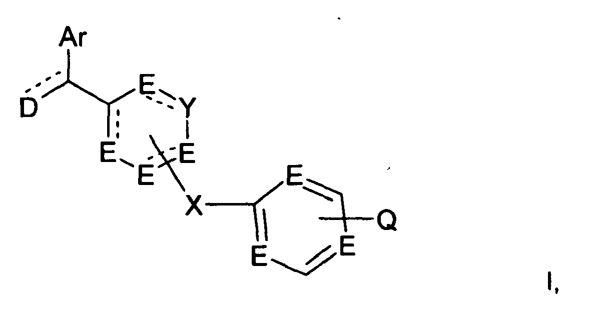 worin
- Ar: einen ein- oder zweikernigen aromatischen Homo- oder He- terocyclus mit 1 bis 4 N-, O- und/oder S-Atomen und 5 bis 10 Gerüstatomen, der unsubstituiert oder ein-, zwei- oder drei- fach durch Carbonylsauerstoff, Hal, A, OH, OA, NH₂, NHA, NA₂, NO₂, CN, OCN, SCN, COOH, COOA, CONH₂, CONHA, CONA₂, NHCOA, NHCONH₂, NHSO₂A, CHO, COA, SO₂NH₂ und/oder S(O)_{g}A substituiert sein kann,
- A: unverzweigtes, verzweigtes oder cyclisches Alkyl mit 1-14 C- Atomen, worin eine oder zwei CH₂-Gruppen durch O- oder S- Atome und/oder durch -CH=CH-Gruppen und/oder auch 1-7 H-Atome durch F und/oder Cl ersetzt sein können,
- Hal: F, Cl, Br oder I,
- D: NH, NH₂, NA₂, NHA, CH₂, CH₃, OH, OA, O oder S,
- E: CH₂, CH, NH oder N,
- Y: E oder eine gesättigte oder ungesättigte Bindung,
- X: CH₂, O oder NH,
- Q: Hal, A, OH, OA, NH₂, NHA, NA₂, NO₂, CN, OCN, SCN, COOH, COOA, CONH₂, CONHA, CONA₂, NHCOA, NHCONH₂, NHSO₂A, CHO, COA, SO₂NH₂ oder X-M,
- M: ein organischer Rest, bestehend aus 2 bis 40 Atomen, von denen mindestens ein Atom weder ein Kohlenstoff- noch ein Wasserstoffatom ist und
- g: 0, 1 oder 2,
- -̅ -̅ -̅ -̅ -̅: eine Einfach- oder Doppelbindung
bedeuten,
sowie ihre pharmazeutisch unbedenklichen Salze, Solvate und Stereoisomere, einschließlich deren Mischungen in allen Verhältnissen.

Es wurde gefunden, dass die beanspruchten Verbindungen der Formel I die Signaltransduktion, die durch Kinasen vermittelt wird, insbesondere durch Tyrosinkinasen, hemmen, regulieren und/oder modulieren können. Insbesondere eignen sich die erfindungsgemäßen Verbindungen als Inhibitoren von Tyrosinkinasen. So können erfindungsgemäße Medikamente und pharmazeutische Zusammensetzungen wirksam zur Behandlung von Krankheiten eingesetzt werden, die durch Kinasen und/oder durch kinase-vermittelte Signaltransduktion verursacht, vermittelt und/oder propagiert werden. Somit eignen sich die erfindungsgemäßen Verbindungen zur Behandlung und Prophylaxe von Krebs, Tumorwachstum, Arteriosklerose, diabetischer Retinopathie, Entzündungserkrankungen, Psoriasis und dergleichen bei Säugetieren.

### Hintergrund der Erfindung

Krebs ist eine Krankheit, deren Ursachen unter anderem in einer gestörten Signaltransduktion zu sehen sind. Insbesondere deregulierte Signaltransduktion über Tyrosinkinasen spielt eine zentrale Rolle beim Wachstum und der Ausbreitung von Krebs (Blume-Jensen, P. und T. Hunter, Nature 411: 355-365, 2001; Hanahan D. und R. A. Weinberg, Cell 100:57-70, 2000). Tyrosinkinasen und insbesondere Rezeptor-Tyrosinkinasen sowie die an sie bindenden Wachstumsfaktoren können so an deregulierter Apoptose, Gewebeinvasion, Metastasierung und allgemein an Signaltransduktionsmechanismen, die zu Krebs führen, beteiligt sein.

Wie bereits erwähnt, ist einer der Hauptmechanismen, durch den die Zellregulation bewirkt wird, die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein sehr weit verbreiteter Prozess in Zellen ist, und da Zellphänotypen größtenteils von der Aktivität dieser Wege beeinflusst werden, wird zur Zeit angenommen, dass eine große Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (siehe Übersichtsartikel: Weinstein-Oppenheimer et al., Pharma. &. Therap. 88:229-279, 2000). Verschiedene Möglichkeiten zur Hemmung, Regulation und Modulation von Kinasen umfassen beispielsweise die Bereitstellung von Antikörpern, antisense-Ribozymen und Inhibitoren. In der Onkologieforschung sind insbesondere Tyrosinkinasen vielversprechende Targets. So sind zahlreiche synthetische kleine Moleküle als Tyrosinkinase-Inhibitoren zur Behandlung von Krebs in der klinischen Entwicklung z.B. Iressa^{®} oder Gleevec^{®}. Allerdings sind hier noch zahlreiche Probleme zu lösen, wie Nebenwirkungen, Dosierung, Resistenz des Tumors, Tumorspezifität und Patientenauswahl.

Bei den Tyrosinkinasen handelt es sich um eine Klasse von Enzymen, die die Übertragung des endständigen Phosphats des Adenosintriphosphats auf Tyrosinreste bei Proteinsubstraten katalysieren. Man nimmt an, dass den Tyrosinkinasen bei verschiedenen Zellfunktionen über die Substratphosphorylierung eine wesentliche Rolle bei der Signaltransduktion zukommt. Obwohl die genauen Mechanismen der Signaltransduktion noch unklar sind, wurde gezeigt, dass die Tyrosinkinasen wichtige Faktoren bei der Zellproliferation, der Karzinogenese und der Zelldifferenzierung darstellen.
Die Tyrosinkinasen lassen sich in Rezeptor-Tyrosinkinasen und zytosolische Tyrosinkinasen einteilen. Die Rezeptor-Tyrosinkinasen weisen einen extrazellulären Teil, einen Transmembranteil und einen intrazellulären Teil auf, während die zytosolischen Tyrosinkinasen ausschließlich intrazellulär vorliegen.

Die Rezeptor-Tyrosinkinasen bestehen aus einer Vielzahl von Transmembranrezeptoren mit unterschiedlicher biologischer Wirksamkeit. So wurden ungefähr 20 verschiedene Unterfamilien von Rezeptor-Tyrosinkinasen identifiziert. Eine Tyrosinkinase-Unterfamilie, die die Bezeichnung EGFR- oder HER-Unterfamilie trägt, besteht aus EGFR, HER2, HER3 und HER4. Zu den Liganden dieser Rezeptor-Unterfamilie zählen der Epithel-Wachstumsfaktor (EGF), der Gewebewachstumsfaktor (TGF-α), Amphiregulin, HB-EGF, Betacellulin und Heregulin. Die Insulin-Unterfamilie, zu der INS-R, IGF-IR und IR-R zählen, stellt eine weitere Unterfamilie dieser Rezeptor-Tyrosinkinasen dar. Die PDGF-Unterfamilie beinhaltet den PDGF-α- and -β-Rezeptor, CSFIR, c-kit und FLK-II. Außerdem gibt es die FLK-Familie, die aus dem Kinaseinsertdomänenrezeptor (KDR) oder VEGFR-2, der fötalen Leberkinase-1 (FLK-1), der fötalen Leberkinase-4 (FLK-4) und der fms-Tyrosinkinase-1 (flt-1) oder VEGFR-1 besteht. Die PDGF- und FLK-Familie werden üblicherweise aufgrund der zwischen den beiden Gruppen bestehenden Ähnlichkeiten in der Gruppe der Splitkinase-Domänen Rezeptor-Tyrosinkinasen zusammengefasst (Laird, A. D. und J. M. Cherrington, Expert. Opin. Investig. Drugs 12(1): 51-64, 2003). Für eine genaue Diskussion der Rezeptor-Tyrosinkinasen siehe die Arbeit von Plowman et al., DN & P 7(6):334-339 (1994).

Die zytosolischen Tyrosinkinasen bestehen ebenfalls aus einer Vielzahl von Unterfamilien, darunter Src, Frk, Btk, Csk, Abl, Zap70, Fes/Fps, Fak, Jak, Ack, and LIMK. Jede dieser Unterfamilien ist weiter in verschiedene Untergruppen unterteilt. So stellt zum Beispiel die Src-Unterfamilie eine der größten Unterfamilien dar. Sie beinhaltet Src, Yes, Fyn, Lyn, Lck, Blk, Hck, Fgr und Yrk. Die Src-Enzymunterfamilie wurde mit der Onkogenese in Verbindung gebracht. Für eine genauere Diskussion der zytosolischen Tyrosinkinasen, siehe die Arbeit von Bolen, Oncogene, 8:2025-2031 (1993). Sowohl die Rezeptor-Tyrosinkinasen als auch die zytosolischen Tyrosinkinasen sind an Signalübertragungswegen der Zelle, die zu Leidenszuständen wie Krebs, Schuppenflechte und Hyperimmunreaktionen führen, beteiligt.

Die vorliegende Erfindung betrifft nun Verbindungen der Formel I gemäß Anspruch 1, vorzugsweise als Regulatoren, Modulatoren oder Inhibitoren von Rezeptor-Tyrosinkinasen der Insulin-Unterfamilie, zu der der Insulinrezeptor IR, der "insulin like growth factor-1 receptor" IGF-1 R und der "insulin related receptor" IRR zählen. Besondere Wirkung zeigen die erfindungsgemäßen Verbindungen bei der Inhibierung der Rezeptor-Tyrosinkinase IGF-1 R.

Wie zuvor erwähnt, gehört der insulinähnliche Wachstumsfaktor-1-Rezeptor (IGF-1 R) zur Familie der transmembranen Tyrosinkinase-Rezeptoren, wie der aus Blutplättchen stammende Wachstumsfaktor-Rezeptor (platelet derived growth factor receptor), der epidermale Wachstumsfaktor-Rezeptor und der Insulinrezeptor. Es gibt zwei bekannte Liganden für den IGF-1R-Rezeptor. Dabei handelt es sich um IGF-1 und IGF-2. Wie hier verwendet, bezieht sich der Begriff "IGF" sowohl auf IGF-1 als auch auf IGF-2. Eine Übersicht über die insulinähnliche Wachstumsfaktor-Familie von Liganden, Rezeptoren und Bindungsproteinen findet sich in Krywicki und Yee, Breast Cancer Research and Treatment, 22:7-19, 1992.

Durch IGF/IGF-1R hervorgerufene Erkrankungen sind durch eine anomale Aktivität oder Hyperaktivität von IGFI/GF-1R gekennzeichnet. Anomale IGF-Aktivität betrifft entweder: (1) IGF- oder IGF-1 R-Expression in Zellen, die gewöhnlich kein IGF oder IGF-1 R exprimieren; (2) erhöhte IGF- oder IGF-1R-Expression, die zu unerwünschter Zellproliferation, wie Krebs, führt; (3) erhöhte IGF- oder IGF-1 R-Aktivität, die zu unerwünschter Zellproliferation, wie Krebs, und/oder zu Hyperaktivität von IGF oder IGF-1R führt. Hyperaktivität von IGF oder IGF 1 R bezieht sich entweder auf eine Amplifikation des Gens, das IGF-1, IGF-2, IGF-1 R codiert, oder die Erzeugung eines Spiegels der IGF-Aktivität, der mit einer Zellproliferationserkrankung korreliert werden kann (d.h. mit steigendem IGF-Spiegel steigt die Schwere eines oder mehrerer Symptome der Zellproliferationserkrankung) die biologische Verfügbarkeit von IGF-1 und IGF-2 kann auch durch das Vorhandensein oder Fehlen eines Satzes von IGF-Bindungsproteinen (IGF-BP's) beeinflusst werden, von denen sechs bekannt sind. Hyperaktivität von IGF/IGF-1R kann auch durch eine Herunterregulation von IGF-2 verursacht werden, das eine IGF-2-Bindungsdomäne, aber keine intrazelluläre Kinasedomäne enthält. Beispiele für durch IGF/IGF-1 R hervorgerufene Erkrankungen umfassen die verschiedenen mit IGF/IGF-1R in Zusammenhang stehenden malignen Erkrankungen beim Menschen, über die Cullen et al., Cancer Investigation, 9(4):443-454, 1991, eine Übersicht geben. Zur klinischen Bedeutung und der Rolle von IGF/IGF-IR bei der Regulation der Osteoblastenfunktion siehe Schmid, Journal of Internal Medicine, 234:535-542, 1993.

Die Aktivitäten von IGF-1R umfassen somit: (1) Phosphorylierung von IGF-1 R-Protein; (2) Phosphorylierung eines IGF-1R-Proteinsubstrats; (3) Wechselwirkung mit einem IGF-Adapterprotein; (4) Oberflächenexpression des IGF-1 R-Proteins. Weitere Aktivitäten des IGF-1R-Proteins können unter Verwendung von Standardtechniken identifiziert werden. Die IGF-1 R-Aktivität kann durch Messen einer oder mehrerer der folgenden Aktivitäten untersucht werden: (1) Phosphorylierung von IGF-1R; (2) Phosphorylierung eines IGF-1R-Substrats; (3) Aktivierung eines IGF-1 R-Adaptermoleküls und (4) Aktivierung stromabwärts gelegener Signalmoleküle und/oder (5) gesteigerte Zellteilung. Diese Aktivitäten können unter Verwendung nachstehend beschriebener und im Stand der Technik bekannter Techniken gemessen werden.

IGF-1 R wurde als essentiell für die Erzeugung und Aufrechterhaltung des transformierten Phänotyps in mehreren Zelltypen in vitro und in vivo angesehen (R. Baserga, Cancer Research 55:249- 252, 1995). Von Herbimycin A wurde gesagt, dass es die IGF-1 R-Protein-Tyrosinkinase und die Zellproliferation in menschlichen Brustkrebszellen hemmt (Sepp-Lorenzino et al., J. Cell Biochem. Suppl. 18b:246, 1994). Experimente zur Untersuchung der Rolle von IGF-1 R bei der Transformation, bei denen Antisense-Strategien, dominant negative Mutationen und Antikörper gegen IGF-1R verwendet wurden, führten zu der Hypothese, dass IGR-1 R ein bevorzugtes Ziel für therapeutische Eingriffe sein kann.

Über seine Rolle bei der Nährstoffzufuhr und bei Diabetes Typ II hinaus, wurde IGF-1 R zudem mit mehreren Krebsarten in Verbindung gebracht. Beispielsweise hat man IGF-1 als autokrinen Wachstumsstimulator bei mehreren Tumorarten, z.B. menschlichen Brustkrebskarzinomzellen (Arteago et al., J. Clin. Invest., 84:1418-1423, 1989) und kleinen Lungentumorzellen (Macauley et al., Cancer Res., 50:2511-2517, 1989) identifiziert. Außerdem scheint IGF-1, der untrennbar mit dem normalen Wachstum und der normalen Differenzierung des Nervensystems verknüpft ist, auch ein autokriner Stimulator menschlicher Gliome zu sein. Sandberg-Nordqvist et al., Cancer Res., 53:2475-2478 (1993).

Ein Beispiel für die potenzielle Beteiligung von IGF-2 an Kolorektalkrebs könnte die Heraufregulation der IGF-2-mRNA in Kolontumoren verglichen mit normalem Dickdarmgewebe sein. (Zhang et al., Science:276: 1268-1272, 1997) IGF-2 kann auch eine Rolle bei der durch Hypoxie verursachenten Neuvaskularisation von Tumoren spielen. (Mines et al., Int. J. Mol. Med. 5:253-259, 2000) IGF-2 kann auch über die Aktivierung einer Insulinrezeptor-Isoform-A eine Rolle bei der Tumorigenese spielen. Die IGF-2-Aktivierung der Insulinrezeptor-Isoform-A aktiviert Signalwege für das Überleben von Zellen, aber das Ausmaß ihres Beitrags zu Tumorzellwachstum und -überleben ist zurzeit noch unbekannt. Die Kinasedomäne der Insulinrezeptor-Isoform-A ist mit derjenigen des Standard-Insulinrezeptors identisch (Scalia et al., J. Cell Biochem. 82:610-618, 2001).

Die Bedeutung von IGF-1R und seiner Liganden in Zelltypen in Kultur (Fibroblasten, Epithelzellen, glatte Muskelzellen, T-Lymphozyten, Myeloidzellen, Chondrozyten und Osteoblasten (die Stammzellen des Knochenmarks)) wird durch die Fähigkeit von IGF-1, Zellwachstum und - proliferation zu stimulieren, demonstriert (Goldring und Goldring, Eukaryotic Gene Expression, 1:301-326, 1991). In einer Reihe neuerer Veröffentlichungen legen Baserga et al. nahe, dass IGF-1 R eine zentrale Rolle beim Mechanismus der Transformation spielt und als solcher ein bevorzugtes Ziel für therapeutische Eingriffe bei einem breiten Spektrum menschlicher maligner Erkrankungen sein könnte (Baserga, Cancer Res., 55:249-252, 1995; Baserga, Cell, 79:927-930, 1994; Coppola et al., Mol. Cell. Biol., 14:4588-4595, 1994; Baserga, Trends in Biotechnology, 14:150-152, 1996; H.M. Khandwala et al., Endocrine Reviews, 21:215-244, 2000).

Die wichtigsten Krebsarten, die unter Verwendung einer erfindungsgemäßen Verbindung behandelt werden können, umfassen Brustkrebs, Prostatakrebs, Kolorektalkrebs, kleinzelligen Lungenkrebs, nicht-kleinzelligen Lungenkrebs, das multiple Myelom sowie das Nierenzellkarzinom und das Endometriumkarzinom.

IGF-1 wurde auch mit der Neovaskularisation der Retina in Verbindung gebracht. Bei einigen Patienten mit hohen IGF-1-Spiegeln wurde eine proliferative Diabetes-Retinopathie beobachtet. (L.E. Smith et al., Nature Medicine, 5:1390-1395, 1999)

Die erfindungsgemäßen Verbindungen können sich aber auch als Mittel zur Alterungsverzögerung eignen. Es wurde beobachtet, dass es eine Verbindung zwischen IGF-Signalen und Alterung gibt. Experimente haben gezeigt, dass Säuger mit kalorienreduzierter Diät niedrige Insulin- und IGF-1-Spiegel aufweisen und eine längere Lebensdauer aufweisen. Ähnliche Beobachtungen wurden auch bei Insekten gemacht (siehe C. Kenyon, Cel 105:165-168, 2001; E. Strauss, Science, 292:41-43, 2001; K.D. Kimura et al., Science, 277:942-946, 1997; M. Tatar et al., Science, 292:107-110, 2001).

Beschrieben wird auch die Verwendung der Verbindungen der Formel I gemäß Anspruch 1 zur Vorbeugung und/oder Behandlung von Erkrankungen im Zusammenhang mit unregulierter oder gestörter Rezeptor-Aktivität. Insbesondere lassen sich die erfindungsgemäßen Verbindungen deshalb bei der Behandlung gewisser Krebsformen einsetzen, wie bspw. Brustkrebs, Prostatakrebs, Darmkrebs, kleinzelliger und nicht-kleinzelliger Lungenkrebs, multiples Myelom, Nierenzellkarzinom oder Korpuskarzinom.

Weiterhin denkbar ist die Verwendung der erfindungsgemäßen Verbindungen zur Behandlung von diabetischer Retinopathie oder zur Verzögerung des Alterungsprozesses. Insbesondere eignen sie sich zur Verwendung in diagnostischen Verfahren zu Erkrankungen im Zusammenhang mit unregulierter oder gestörter IGF-1 R-Aktivität.

Zudem können die erfindungsgemäßen Verbindungen verwendet werden, um bei gewissen existierenden Krebs-Chemotherapien und -bestrahlungen additive oder synergistische Effekte zu erzielen und/oder, um die Wirksamkeit gewisser existierender Krebs-Chemotherapien und -bestrahlungen wiederherzustellen.

Eine Reihe von aza-heterozyklischen Verbindungen wurden bisher als Kinase-Inhibitoren beschrieben, etwa in der WO 03/018021, WO 03/018021 oder der WO 04/056807.

DE 2 142 353 offenbart 2-Hydroxy-4'-phenoxybenzophenonverbindungen und deren Verwendung als Stabilisatoren für organische Materialien.

DE 2 847 662 A1 offenbart ein Verfahren zur Herstellung von Hydroxydiarylethern, die als Zwischenprodukte bei der Herstellung von Verbindungen mit herbizider und pflanzenregulatorischer Wirkung Verwendung finden können.

Holt et al. offenbart Benzophenon- und Indolcarbonsäuren und deren Wirkung als potente Type-2 spezifische Inhibitoren der menschlichen Steroid 5α-Reduktase (JOURNAL OF MEDICINAL CHEMISTRY, Bd. 38, Nr. 1, 1995, Seiten 13-15, XP002384662).

Cinque et al. offenbart verschiedene Verbindungen mit 4-Phenoxyphenoxy-Struktur und deren wachstumshemmende Wirkung gegenüber dem Krankheitserreger Trypanosoma cruzi (JOURNAL OF MEDICINAL CHEMISTRY, Bd. 41, Nr. 9, 1998, Seiten 1540-1554). Sarmiento et al. offenbart verschiedene Verbindungen mit unterschiedlicher Struktur und deren Wirkung als Inhibitoren der Protein-Thyrosin-Phosphatase 1 B. (JOURNAL OF MEDICINAL CHEMISTRY, Bd. 43, Nr. 2, 2000, Seiten 146-155).

Der Erfindung lag daher die Aufgabe zugrunde, neue Verbindungen mit vorteilhaften therapeutischen Eigenschaften aufzufinden, die zur Herstellung von Arzneimitteln verwendet werden können.
So ist die Identifikation und Bereitstellung von chemischen Verbindungen, die die Signaltransduktion der Tyrosinkinasen spezifisch hemmen, regulieren und/oder modulieren, wünschenswert und daher ein Ziel der vorliegenden Erfindung.

### Beschreibung der Erfindung

Es wurde gefunden, dass die Verbindungen der Formel I gemäß Anspruch 1 und ihre Salze bei guter Verträglichkeit sehr wertvolle pharmakologische Eigenschaften besitzen. Insbesondere wurde gefunden, dass die erfindungsgegenständlichen Verbindungen der Formel I gemäß Anspruch 1 überraschenderweise wirksame Kinase-Inhibitoren darstellen, wobei sie insbesondere eine Tyrosinkinasen-inhibierende Wirkung, und in besonderem Maße eine IGF-R1 inhibierende Wirkung zeigen.

Generell gilt, dass sämtliche Reste, die mehrfach auftreten, gleich oder verschieden sein können, d.h. unabhängig voneinander sind. Vor- und nachstehend haben die Reste bzw. Parameter die für die Formel I angegebenen Bedeutungen, falls nicht ausdrücklich etwas anderes angegeben ist.
Dementsprechend sind Gegenstand der Erfindung insbesondere diejenigen Verbindungen der Formel I gemäß Anspruch 1, in denen mindestens einer der genannten Reste eine der nachstehend angegebenen bevorzugten Bedeutungen hat.

Hal bedeutet Fluor, Chlor, Brom oder Iod, insbesondere Fluor oder Chlor.

A bedeutet Alkyl, ist unverzweigt (linear), verzweigt oder cyclisch, und hat 1, 2, 3, 4, 5, 6, 7, 8, 9, 10, 11, 12, 13 oder 14 C-Atome.

A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1-oder 2-Ethylbutyl, 1-Ethyl-1-methylpropyl, 1-Ethyl-2-methylpropyl, 1,1,2-oder 1,2,2-Trimethylpropyl, lineares oder verzweigtes Heptyl, Octyl, Nonyl oder Decyl.

A bedeutet ganz besonders bevorzugt Alkyl mit 1, 2, 3, 4, 5 oder 6 C-Atomen, worin eine oder zwei CH₂-Gruppen durch O- oder S-Atome und/oder durch -CH=CH-Gruppen und/oder auch 1-7 H-Atome durch F und/oder Cl ersetzt sein können, wie z.B. Methyl, Ethyl, Propyl, Isopropyl, Butyl, Isobutyl, sek.-Butyl, tert.-Butyl, Pentyl, Hexyl, Trifluormethyl, Pentafluorethyl, 1,1-Difluormethyl oder 1,1,1-Trifluorethyl.

Cycloalkyl bedeutet vorzugsweise Cyclopropyl, Cyclobutyl, Cylopentyl, Cyclohexyl oder Cycloheptyl.

OA ist vorzugsweise Methoxy, ferner auch Ethoxy, n-Propoxy, Isopropoxy, n-Butoxy, Isobutoxy, sek.-Butoxy oder tert.-Butoxy.

Ar bedeutet z.B. unsubstituiertes Phenyl, Naphthyl oder Biphenyl, weiterhin vorzugsweise z.B. durch A, Fluor, Chlor, Brom, Iod, Hydroxy, Methoxy, Ethoxy, Propoxy, Butoxy, Pentyloxy, Hexyloxy, Nitro, Cyan, Formyl, Acetyl, Propionyl, Trifluormethyl, Amino, Methylamino, Ethylamino, Dimethylamino, Diethylamino, Benzyloxy, Sulfonamido, Methylsulfonamido, Ethylsulfonamido, Propylsulfonamido, Butylsulfonamido, Dimethylsulfonamido, Phenylsulfonamido, Carboxy, Methoxycarbonyl, Ethoxycarbonyl, Aminocarbonyl mono-, di- oder trisubstituiertes Phenyl, Naphthyl oder Biphenyl.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m-oder p-Aminophenyl, o-, m- oder p-(N-Methylamino)-phenyl, o-, m- oder p-(N-Methylaminocarbonyl)-phenyl, o-, m- oder p-Acetamidophenyl, o-, m-oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m-oder p-Bromphenyl, o-, m- oder p- Chlorphenyl, o-, m- oder p-(Methylsulfonamido)-phenyl, o-, m- oder p-(Methylsulfonyl)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-Iodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet weiterhin vorzugsweise unsubstituiertes oder ein-, zwei- oder dreifach z.B. durch Carbonylsauerstoff, F, Cl, Br, Methyl, Ethyl, 1-Propyl, 2-Propyl, 1-Butyl, 2-Butyl, t-Butyl, Phenyl, Benzyl, -CH₂-Cyclohexyl, Hydroxy, Methoxy, Ethoxy, Amino, Methylamino, Dimethylamino, Nitro, Cyan, Carboxy, Methoxycarbonyl, Aminocarbonyl, Methylaminocarbonyl, Dimethylaminocarbonyl, Acetamino, Ureido, Methylsulfonylamino, Formyl, Acetyl, Aminosulfonyl und/oder Methylsulfonyl substituiertes 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 2-, 3-, 4- oder 5-Isoindolyl, 2-, 6, -oder 8-Purinyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6-oder 7-Benzisothiazolyl, 4-, 5-, 6- oder 7-Benz-2,1,3-oxadiazolyl, 1-, 3-, 4-, 5-, 6-, 7- oder 8-Isochinolyl, 3-, 4-, 5-, 6-, 7- oder 8-Cinolinyl, 2-, 4-, 5-, 6-, 7- oder 8-Chinazolinyl, 5- oder 6-Chinoxalinyl, 4-, 5-, oder 6-Phthalazinyl, 2-, 3-, 5-, 6-, 7- oder 8-2H-Benzo[1,4]oxazinyl, weiter bevorzugt 1,3-Benzo-dioxol-5-yl, 1,4-Benzodioxan-6-yl, 2,1,3-Benzothiadiazol-4- oder -5-yl oder 2,1,3-Benzoxadiazol-5-yl.

Die heterocyclischen Reste können auch teilweise oder vollständig hydriert sein und bedeuten z.B. auch 2,3-Dihydro-2-, -3-, -4- oder -5-furyl, 2,5-Dihydro-2-, -3-, -4- oder -5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder-4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Die Bezeichnung "substituiert" bezieht sich vorzugsweise auf die Substitution mit den obengenannten Substituenten, wobei mehrere unterschiedliche Substitutionsgrade möglich sind, falls nicht anders angegeben.

Erfindungsgemäß sind auch alle physiologisch unbedenklichen Salze, Solvate und Stereoisomere der Verbindungen gemäß Anspruch 1, einschließlich deren Mischungen in allen Verhältnissen.

Die Verbindungen der Formel I können ein oder mehrere chirale Zentren aufweisen. Sie können dementsprechend in verschiedenen enantiomeren Formen auftreten und in racemischer oder in optisch aktiver Form vorliegen. Gegenstand der Erfindung sind deshalb auch die optisch aktiven Formen (Stereoisomeren), die Enantiomeren, die Racemate, die Diastereomeren sowie Hydrate und Solvate dieser Verbindungen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktive Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wässrige oder alkoholische Lösungsmittelgemische wie z.B. Hexan/Isopropanol/ Acetonitril z.B. im Verhältnis 82:15:3.
Eine elegante Methode zur Spaltung von Racematen mit Estergruppen (z.B. Acetylester) stellt die Verwendung von Enzymen, insbesondere Esterasen, dar.

Erfindungsgemäß sind Verbindungen, ausgewählt aus den in der Tabelle 1 aufgeführten Verbindungen sowie ihre pharmazeutisch unbedenklichen Salze, Solvate und Stereoisomere, einschließlich deren Mischungen in allen Verhältnissen.

**Tabelle 1**

| | IC50 (µM) | Schmelzpunkt |
|---|---|---|
| 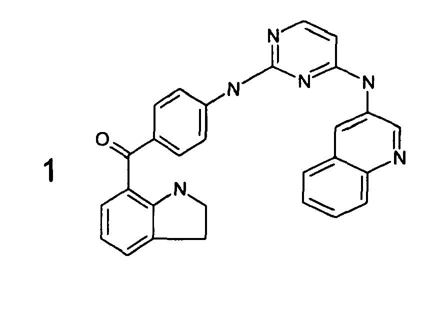 | 1,11 | 235-238°C |
| 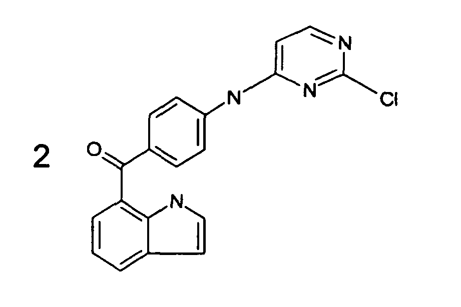 | 2,6 | 196-197°C (Trifluoracetat) |
| 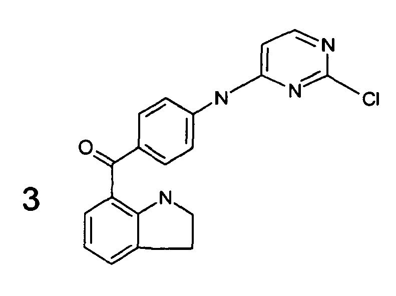 | 7,85 | |
| 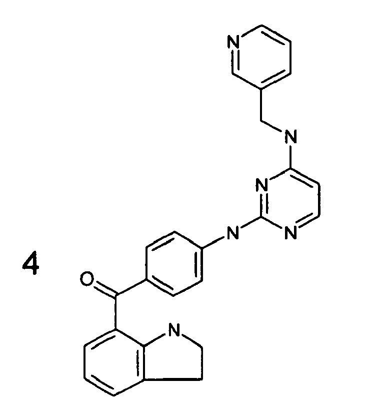 | 2,4 | 80-81°C |
| 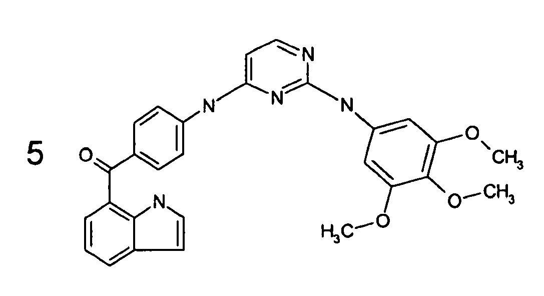 | 2,2 | 115-116°C |
| 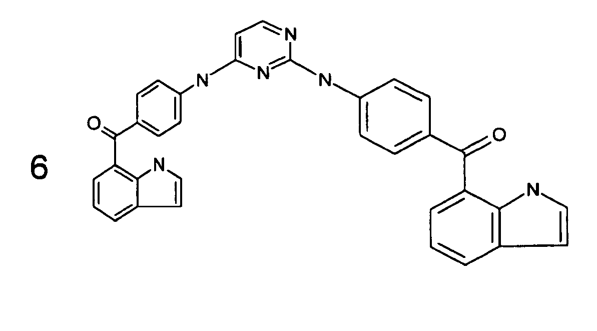 | 1,8 | 150°C Zersetzung |
| 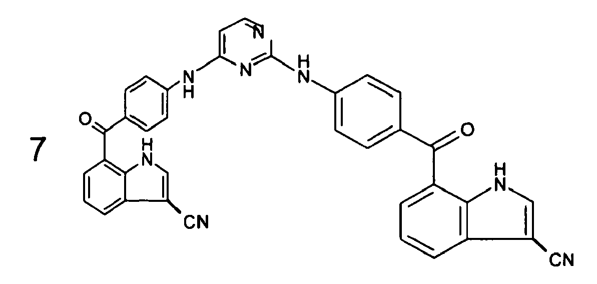 | 0,49 | |
| 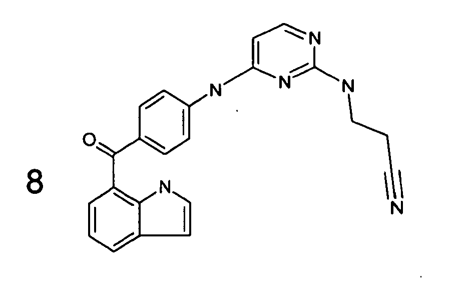 | 3,1 | 204-206°C (Hydrochlorid) |
| 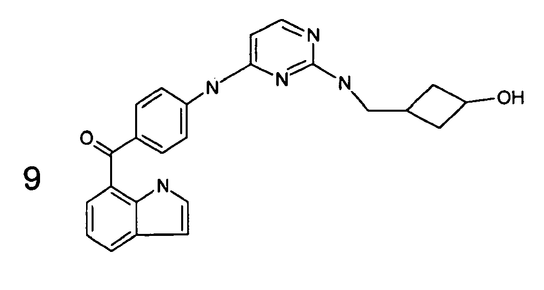 | 1,2 | 115-120°C (Hydrochlorid) |
| 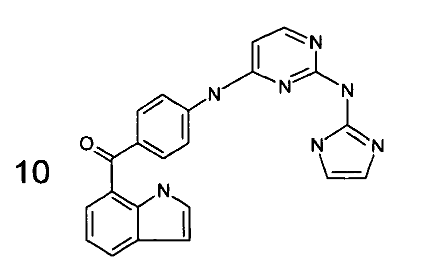 | 0,15 | 202-207°C (Hydrochlorid) |
| 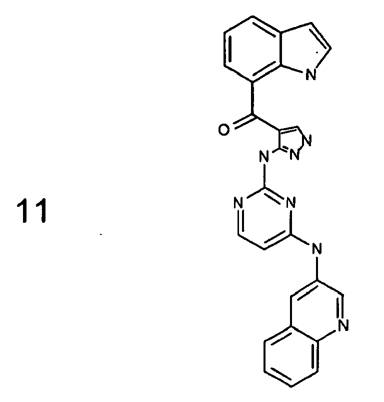 | 0,63 | |
| 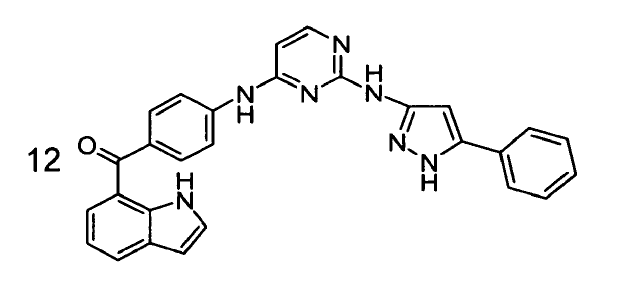 | 10 | 166-166,5°C (Trifluoracetat) |
| 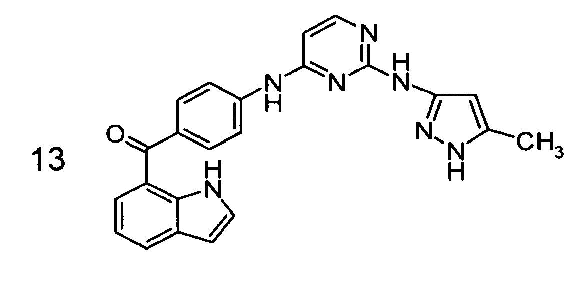 | 2,9 | 117-119°C (Trifluoracetat) |
| 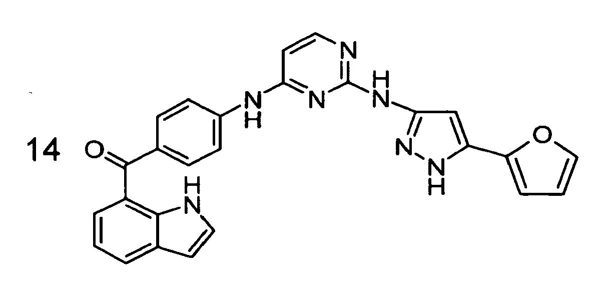 | 15 | 171-172°C (Trifluoracetat) |
| 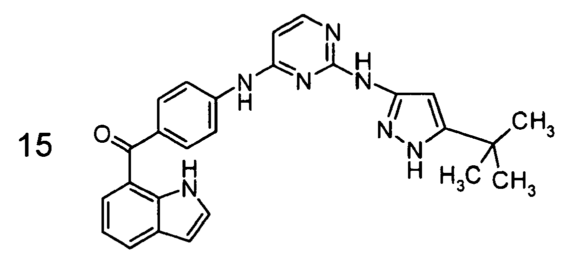 | 12 | 140-142°C (Trifluoracetat) |
| 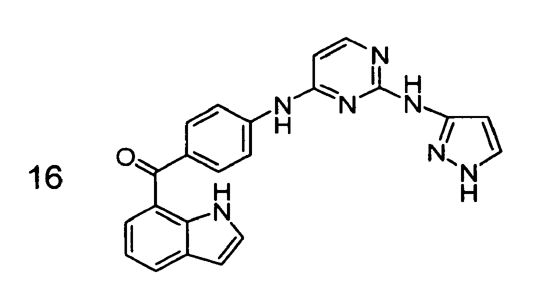 | 2,4 | 198-200°C (Trifluoracetat) |

Als Säureadditionssalze kommen anorganische oder organische Salze aller physiologisch oder pharmakologisch unbedenklichen Säuren in Frage, beispielsweise Halogenide, insbesondere Hydrochloride oder Hydrobromide, Lactate, Sulfate, Citrate, Tartrate, Maleate, Fumarate, Oxalate, Acetate, Phosphate, Methylsulfonate oder p-Toluolsulfonate.

Unter Solvaten der Verbindungen der Formel I werden Anlagerungen von inerten Lösungsmittelmolekülen an die Verbindungen der Formel I verstanden, die sich aufgrund ihrer gegenseitigen Anziehungskraft ausbilden. Solvate sind beispielsweise Hydrate, wie Monohydrate oder Dihydrate oder Alkoholate, d.h. Additionsverbindungen mit Alkoholen wie beispielsweise mit Methanol oder Ethanol.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder angestrebt wird.

Darüber hinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder Verhinderung von Nebenwirkungen oder auch die Verminderung des Fortschreitens einer Krankheit, eines Leidens oder einer Störung. Die Bezeichnung "therapeutisch wirksame Menge" umfasst auch die Mengen, die wirkungsvoll sind, die normale physiologische Funktion zu erhöhen.

Gegenstand der Erfindung sind auch Mischungen der erfindungsgemäßen Verbindungen der Formel I gemäß Anspruch 1, z.B. Gemische zweier Diastereomere z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Die Verbindungen der Formel I sowie ihrer physiologisch unbedenklichen Salze, Solvate und Stereoisomere können durch ein Verfahren hergestellt werden, das dadurch gekennzeichnet ist, dass man eine Verbindung der Formel V, 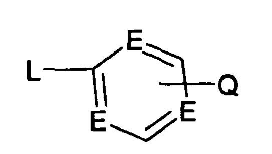 worin E und Q die oben angegebenen Bedeutungen haben und L eine Abgangsgruppe wie bspw. Cl, Br, I, Mesylat, Tosylat, Phenylsulfonat oder Trifluoracetat, mit einer Verbindung der Formel IV, 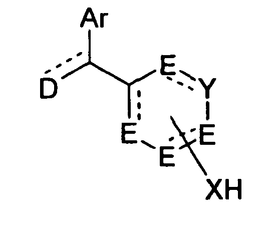 worin E, X, Y, Ar und D die oben angegebenen Bedeutungen haben, umsetzt und/oder eine Base oder Säure der Formel I in eines ihrer Salze umwandelt.

Die Verbindungen der Formel V und IV sind in der Regel bekannt. Sind sie neu, so können sie nach an sich bekannten Methoden hergestellt werden, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York) beschrieben sind.

Die Verbindungen der Formel I und auch die Ausgangsstoffe zu ihrer Herstellung werden nach an sich bekannten Methoden hergestellt, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das Verfahren können auch in situ gebildet werden, derart, dass man sie aus dem Reaktionsgemisch nicht isoliert, sondern sofort weiter zu den Verbindungen der Formel I umsetzt. Andererseits ist es möglich, die Reaktion stufenweise durchzuführen.

Die Aza-Heterocyclen der Formel I können vorzugsweise erhalten werden, indem man ein Edukt der Formel V mit einem Edukt der Formel IV wie folgt umsetzt:
Eine Verbindung der Formel V wird zusammen mit einer Verbindung der Formel IV in einem inerten Lösungsmittel gelöst und anschließend bei erhöhter Temperatur gerührt. Anschließend wird das Reaktionsgemisch aufgereinigt und das Produkt als Feststoff, vorzugsweise kristallin, isoliert.

Die Edukte der Formeln V und IV sind in der Regel bekannt und kommerziell erhältlich; die nicht bekannten Verbindungen der Formeln V und IV können leicht analog zu bekannten Verbindungen hergestellt werden. Die Herstellung der Verbindung der Formel V (2-Chloro-pyrimidin-4-yl)-quinolin-3-yl-amin und der Verbindung der Formel IV (4-Amino-phenyl)-(2,3-dihydro-1H-indol-7-yl)-methanon sind in den Beispielen 1 und 2 beschrieben, die Herstellung von (2,3-Dihydro-1H-indol-7-yl)-[4-[4-(quinolin-3ylamino)-pyrimidin-2-ylamino]-phenyl}-methanon im Beispiel 3.

Die zuvor beschriebene Umsetzung erfolgt in der Regel in einem inerten Lösungsmittel. Als inerte Lösungsmittel für die zuvor beschriebenen Umsetzungen eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Ether wie Diethylether, Diisopropylether, Tetrahydrofuran (THF) oder Dioxan; Glykolether wie Ethylenglykolmonomethyl- oder monoethylether (Methylglykol oder Ethylglykol), Ethylenglykoldimethylether (Diglyme); Ketone wie Aceton oder Butanon; Amide wie Acetamid, N-Methylpyrrolidon (NMP), Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel. Bevorzugt sind Sulfoxide wie Dimethylsulfoxid (DMSO).

Die Menge des Lösungsmittels ist nicht kritisch, vorzugsweise können 5 g bis 500 g Lösungsmittel je g der zu bildenden Verbindung der Formel I zugesetzt werden.

In der Regel wird bei einem Druck von 1 bis 200 bar gearbeitet, bevorzugt jedoch bei Normaldruck.

Die Reaktionstemperatur für die zuvor beschriebenen Umsetzungen liegt je nach den angewendeten Bedingungen zwischen etwa -10° und 200°, normalerweise zwischen 60° und 180°, bevorzugt zwischen 80° und 120°. Die Reaktionszeit liegt je nach den angewendeten Bedingungen zwischen einigen Minuten und mehreren Tagen, vorzugsweise im Bereich von mehreren Stunden.

Die Reaktion kann auch in heterogener Phase ausgeführt werden, wobei vorzugsweise eine wässrige Phase und eine Benzol- oder Toluol-Phase verwendet werden. Hier kommt ein Phasentransfer-Katalysator zum Einsatz, wie beispielsweise Tetrabutylammoniumiodid und gegebenenfalls ein Acylierungskatalysator, wie beispielsweise Dimethylaminopyridin.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure; Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen.

Verbindungen der Formel I können ferner erhalten werden, indem man sie aus einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel I entspreche, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entspreche, jedoch anstelle einer Gruppe -COOH eine Gruppe -COOR" tragen, worin R" eine Hydroxyschutzgruppe bedeutet.
Bevorzugte Ausgangsstoffe sind auch die Oxadiazolderivate, die in die entsprechenden Amidinoverbindungen überführt werden können.

Es können auch mehrere - gleiche oder verschiedene - geschützte Amino-und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Tolyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Ferner kann man freie Aminogruppen in üblicher Weise mit einem Säurechlorid oder -anhydrid acylieren oder mit einem unsubstituierten oder substituierten Alkylhalogenid alkylieren, oder mit CH3-C(=NH)-OEt umsetzen, zweckmäßig in einem inerten Lösungsmittel wie Dichlormethan oder THF und /oder in Gegenwart einer Base wie Triethylamin oder Pyridin bei Temperaturen zwischen -60 und +30°.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkyl- oder Silylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, 4-Methoxybenzyl, p-Nitrobenzoyl, p-Toluolsulfonyl, tert.-Butyl und Acetyl, wobei Benzyl und tert.-Butyl besonders bevorzugt sind.

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°C, vorzugsweise arbeitet man zwischen 15 und 30°C (Raumtemperatur, RT).

Die Gruppen BOC, OBut und Mtr können z. B. bevorzugt mit TFA in Dichlormethan oder mit etwa 3 bis 5n HCl in Dioxan bei 15-30°C abgespalten werden, die FMOC-Gruppe mit einer etwa 5- bis 50 %igen Lösung von Dimethylamin, Diethylamin oder Piperidin in DMF bei 15-30° C.

Hydrogenolytisch entfernbare Schutzgruppen (z. B. CBZ, Benzyl oder die Freisetzung der Amidinogruppe aus ihrem Oxadiazolderivat) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Ester können z.B. mit Essigsäure oder mit NaOH oder KOH in Wasser, Wasser-THF oder Wasser-Dioxan bei Temperaturen zwischen 0 und 100° verseift werden.

Weitere Methoden zur Entfernung von Schutzgruppen ist beispielsweise in Theodora W. Green, Peter G. M. Wuts: Protective Groups in Organic Synthesis, 3rd Edition John Wiley & Sons (1999) beschrieben.

Erfindungsgemäße Verbindungen der Formel I gemäß Anspruch 1 können aufgrund ihrer Molekülstruktur chiral sein und dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische, biochemische oder physikalische Maßnahmen, aufgetrennt oder bereits als solche bei der Synthese eingesetzt werden.

Durch übliche Aufarbeitungsschritte wie z.B. Wasserzugabe zum Reaktionsgemisch und Extraktion können die Verbindungen der Formel I nach Entfernung des Lösungsmittels erhalten werden. Es kann vorteilhaft sein, zur weiteren Reinigung des Produktes eine Destillation oder Kristallisation anzuschließen.

Ein weiterer Gegenstand der Erfindung sind Arzneimittel, enthaltend wenigstens eine erfindungsgemäße Verbindung gemäß Anspruch 1 und/oder ihre physiologisch unbedenklichen Salze, Solvate und Stereoisomere, einschließlich deren Mischungen in allen Verhältnissen.
Weiterhin kann eine erfindungsgemäße pharmazeutische Zubereitung, weitere Träger- und/oder Hilfsstoffe sowie gegebenenfalls einen oder mehrere weitere Arzneimittelwirkstoffe enthalten.

Gegenstand der Erfindung ist ferner ein Verfahren zur Herstellung eines Arzneimittels, dadurch gekennzeichnet, dass man eine erfindungsgemäße Verbindung gemäß Anspruch 1 und/oder eines ihrer physiologisch unbedenklichen Salze, Derivate, Solvate und Stereoisomere, einschließlich deren Mischungen in allen Verhältnissen zusammen mit einem festen, flüssigen oder halbflüssigen Träger- oder Hilfsstoff in eine geeignete Dosierungsform bringt.

Gegenstand der Erfindung ist auch ein Set (Kit) bestehend aus getrennten Packungen von
a) einer wirksamen Menge einer erfindungsgemäßen Verbindung gemäß Anspruch 1 und/oder ihrer physiologisch unbedenklichen Salze, Derivate, Solvate und Stereoisomere, einschließlich deren Mischungen in allen Verhältnissen und
b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer erfindungsgemäßen Verbindung und/oder ihrer pharmazeutisch verwendbaren Derivate, Solvate und Stereoisomere, einschließlich deren Mischungen in allen Verhältnissen, und einer wirksamen Menge eines weiteren Arzneimittelwirkstoffs gelöst oder in lyophylisierter Form vorliegt.

Arzneimittel können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Geschlecht, Gewicht und Zustand des Patienten. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche Arzneimitel mit einem der im pharmazeutischen Fachgebiet allgemein bekannten Verfahren herstellen.

Arzneimittel lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Arzneimittel können mit allen im pharmazeutischen Fachgebiet bekannten Verfahren hergestellt werden, indem beispielsweise der Wirkstoff mit dem bzw. den Trägerstoff(en) oder Hilfsstoff(en) zusa m-mengebracht wird.

An die orale Verabreichung angepasste Arzneimittel können als separate Einheiten, wie z.B. Kapseln oder Tabletten; Pulver oder Granulate; Lösungen oder Suspensionen in wässrigen oder nichtwässrigen Flüssigkeiten; essbare Schäume oder Schaumspeisen; oder Öl-in-Wasser-Flüssigemulsionen oder Wasser-in-Öl-Flüssigemulsionen dargereicht werden.

So lässt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem essbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit-und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpresst wird, ein Schmiermittel und ein Sprengmittel zugegeben werden und das Ganze zu Tabletten verpresst wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch lässt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepresst wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengussformen zu verhindern. Das gefettete Gemisch wird dann zu Tabletten verpresst. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpresst werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so dass eine gegebene Quantität eine vorgegebene Menge der Verbindung enthält. Sirupe lassen sich herstellen, indem die Verbindung in einer wässrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung lässt sich auch so herstellen, dass die Freisetzung verlängert oder retardiert wird, wie beispielsweise durch Beschichtung oder Einbettung von partikulärem Material in Polymere, Wachs u.ä.

Die erfindungsgemäßen Verbindungen sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Verbindungen sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspart amidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsi-Ion-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepasste Arzneimittel können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6):318 (1986) allgemein beschrieben.

An die topische Verabreichung angepasste Arzneimittel können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepassten Arzneimittel gehören Augentropfen, wobei der Wirkstoff in einem geeigneten Träger, insbesondere einem wässrigen Lösungsmittel, gelöst oder suspendiert ist.

An die topische Applikation im Mund angepasste Arzneimittel umfassen Lutschtabletten, Pastillen und Mundspülmittel.

An die rektale Verabreichung angepasste Arzneimittel können in Form von Zäpfchen oder Einläufen dargereicht werden.

An die nasale Verabreichung angepasste Arzneimittel in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepasste Arzneimittel umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepasste Arzneimittel können als Pessare, Tampons, Cremes, Gele, Pasten, Schäume oder Sprayformulierungen dargereicht werden.

Zu den an die parenterale Verabreichung angepassten Arzneimittel gehören wässrige und nichtwässrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wässrige und nichtwässrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so dass nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, dass die erfindungsgemäßen Arzneimittel neben den obigen besonders erwähnten Bestandteilen andere im Fachgebiet übliche Mittel mit Bezug auf die jeweilige Art der pharmazeutischen Formulierung enthalten können; so können beispielsweise für die orale Verabreichung geeignete Arzneimittel Geschmacksstoffe enthalten.

Eine therapeutisch wirksame Menge einer Verbindung der vorliegenden Erfindung hängt von einer Reihe von Faktoren ab, einschließlich z.B. dem Alter und Gewicht des Empfängers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer Verbindung der Formel I für die Behandlung der erfindungsgemäßen Erkrankungen im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so dass die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung per se bestimmt werden.

Die erfindungsgemäßen Verbindungen zeigen eine vorteilhafte biologische Aktivität, die in Enzym-Assays leicht nachweisbar ist. In derartigen auf Enzymen basierenden Assays zeigen und bewirken die erfindungsgemäßen Verbindungen bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Gegenstand der vorliegenden Erfindung sind erfindungsgemäße Verbindungen gemäß Anspruch 1 als Effektoren, bevorzugt als Inhibitoren der hierin beschriebenen Signalwege. Besonders bevorzugter Gegenstand der Erfindung sind deshalb erfindungsgemäße Verbindungen als Aktivatoren und Inhibitoren von Tyrosinkinasen, bevorzugt als Inhibitoren von Rezeptor-Tyrosinkinasen, insbesondere der Insulin-Unterfamilie, zu der INS-R, IGF-IR und IR-R zählen. Hierbei zeigen die erfindungsgemäßen Verbindungen gemäß Anspruch 1 eine besondere Wirkung bei der Inhibierung der Rezeptortyrosinkinase IGF-1 R.

Wie vorstehend besprochen, sind die durch die erfindungsgemäßen Verbindungen beeinflussten Signalwege für verschiedene Erkrankungen relevant. Dementsprechend sind die erfindungsgemäßen Verbindungen nützlich bei der Prophylaxe und/oder Behandlung von Erkrankungen, die von den genannten Signalwegen durch Interaktion mit einem oder mehreren der genannten Signalwege abhängig sind.

Ein weiterer Gegenstand der vorliegenden Erfindung ist deshalb die Verwendung von erfindungsgemäßen Verbindungen gemäß Anspruch 1 und/oder ihrer physiologisch unbedenklichen Salze, Solvate und Stereoisomere, einschließlich deren Mischungen in allen Verhältnissen zur Herstellung eines Medikaments zur Behandlung und/oder Prophylaxe von Krankheiten, insbesondere solcher Krankheiten, die durch Kinasen und/oder durch kinase-vermittelte Signaltransduktion verursacht, vermittelt und/oder propagiert werden. Bevorzugt sind hierbei Tyrosinkinasen, ausgewählt aus der Gruppe der Rezeptor-Tyrosinkinasen. Besonders bevorzugt handelt es sich dabei um IGF-1 R.

Außerdem eignen sich die vorliegenden Verbindungen als pharmazeutische Wirkstoffe für Säugetiere, insbesondere für den Menschen, bei der Behandlung von tyrosinkinasebedingten Krankheiten. Der Ausdruck "tyrosinkinasebedingte Krankheiten " bezieht sich auf pathologische Zustände, die von der Aktivität einer oder mehrerer Tyrosinkinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen Krebs, Tumorwachstum, Arteriosklerose, diabetischer Retinopathie und Entzündungserkrankungen.

Gewöhnlich werden die hier besprochenen Erkrankungen in zwei Gruppen eingeteilt, in hyperproliferative und nicht-hyperproliferative Erkrankungen. In diesem Zusammenhang werden Psoriasis, Arthritis, Entzündungen, Endometriose, Vernarbung, gutartige Prostatahyperplasie, immunologische Krankheiten, Autoimmunkrankheiten und Immunschwächekrankheiten als nicht-krebsartige Krankheiten angesehen, von denen Arthritis, Entzündung, immunologische Krankheiten, Autoimmunkrankheiten und Immunschwächekrankheiten gewöhnlich als nicht-hyperproliferative Erkrankungen angesehen werden.

In diesem Zusammenhang sind Hirnkrebs, Lungenkrebs, Plattenepithelkrebs, Blasenkrebs, Magenkrebs, Pankreaskrebs, Leberkrebs, Nierenkrebs, Darmkrebs, Brustkrebs, Kopfkrebs, Halskrebs, Ösophaguskrebs, gynäkologischer Krebs, Schilddrüsenkrebs, Lymphome, chronische Leukämie und akute Leukämie als krebsartige Erkrankungen anzusehen, die alle gewöhnlich zur Gruppe der hyperproliferative Erkrankungen gezählt werden. Insbesondere krebsartiges Zellwachstum und insbesondere durch IGF-1 R direkt oder indirekt vermitteltes krebsartiges Zellwachstum ist eine Erkrankung, die ein Ziel der vorliegenden Erfindung darstellt.

Gegenstand der vorliegenden Erfindung ist deshalb die Verwendung von erfindungsgemäßen Verbindungen gemäß Anspruch 1 zur Herstellung eines Medikaments für die Behandlung und/oder Prophylaxe der genannten Erkrankungen sowie auch ein Verfahren zur Behandlung der genannten Erkrankungen, umfassend die Verabreichung eines oder mehrerer erfindungsgemäßer Verbindungen an einen Patienten mit Bedarf an einer derartigen Verabreichung.

Der Empfänger oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Empfänglichkeit einer bestimmten Zelle gegenüber der Behandlung mit den erfindungsgemäßen Verbindungen kann durch *in vitro*-Tests bestimmt werden. Typischerweise wird eine Kultur der Zelle mit einer erfindungsgemäßen Verbindung bei verschiedenen Konzentrationen für eine Zeitdauer inkubiert, die ausreicht, um den Wirkstoffen zu ermöglichen, Zelltod zu induzieren oder Migration zu inhibieren, gewöhnlich zwischen ungefähr einer Stunde und einer Woche. Zu *in vitro*-Tests können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der spezifischen Zellzahl und kann fortgesetzt werden, bis im Wesentlichen keine unerwünschten Zellen mehr im Körper nachgewiesen werden.

Zur Identifikation von Kinase-Inhibitoren stehen verschiedene Assay-Systeme zur Verfügung. Beim Scintillation-Proximity-Assay (Sorg et al., J. of. Biomolecular Screening:7, 11-19, 2002) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 191-214, 2002).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., Biochem. J. 366:977-981, 2002).

Es gibt viele mit einer Deregulation der Zellproliferation und des Zelltods (Apoptose) einhergehende Erkrankungen und Krankheitszustände. Die Erkrankungen und Krankheitszustände die durch erfindungsgemäße Verbindungen behandelt, verhindert oder gelindert werden können umfassen die nachfolgend aufgeführten Erkrankungen und Krankheitszustände, sind jedoch nicht darauf beschränkt. Die erfindungsgemäßen Verbindungen sind nützlich bei der Behandlung und/oder Prophylaxe einer Reihe verschiedener Erkrankungen und Krankheitszustände, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

Die vorliegende Erfindung beschreibt die Verwendung der erfindungsgemäßen Verbindungen gemäß Anspruch 1 zur Behandlung oder Vorbeugung von Krebs. Gegenstand der Erfindung ist insbesondere die Verwendung von erfindungsgemäßen Verbindungen gemäß Anspruch 1 und/oder ihrer physiologisch unbedenklichen Salze, Solvate und Stereoisomere, einschließlich deren Mischungen in allen Verhältnissen zur Herstellung eines Medikaments zur Behandlung und/oder Prophylaxe von festen Tumoren, wobei der feste Tumor besonders bevorzugt aus der Gruppe bestehend aus Gehirntumor, Tumor des Urogenitaltrakts, Tumor des lymphatischen Systems, Magentumor, Kehlkopftumor, Lungentumor ausgewählt ist. Bevorzugt können auch feste Tumore ausgewählt aus der Gruppe bestehend aus Monozytenleukämie, Lungenadenokarzinom, kleinzellige und nicht- kleinzellige Lungenkarzinome, Nierenzellkarzinom, Endometriumkarzinom, multiples Myelom, Prostatakrebs, Kolorektalkrebs, Bauchspeicheldrüsenkrebs, Glioblastome und Brustkarzinom mit Medikamenten enthaltend erfindungsgemäße Verbindungen behandelt werden.

Die erfindungsgemäßen Verbindungen können an Patienten zur Behandlung von Krebs verabreicht werden. Die vorliegenden Verbindungen hemmen über die Bindung an IGF-1R die Tumorangiogenese und beeinflussen so das Wachstum von Tumoren (S.E. Dunn et al. Mol Carcinog. 2000 Jan;27(1):10-7). Die Eigenschaften der erfindungsgemäßen Verbindungen lassen diese auch für die Behandlung bestimmter Formen von Blindheit, die mit Retina-Gefäßneubildung in Zusammenhang stehen, geeignet erscheinen.

Gegenstand der Erfindung ist deshalb auch die Verwendung von erfindungsgemäßen Verbindungen gemäß Anspruch 1 und/oder ihrer physiologisch unbedenklichen Salze, Solvate und Stereoisomere, einschließlich deren Mischungen in allen Verhältnissen zur Herstellung eines Medikaments zur Behandlung und oder Prophylaxe von Krankheiten, die durch Angiogenese verursacht, vermittelt und/oder propagiert werden.

Eine derartige Krankheit, an der Angiogenese beteiligt ist, ist eine Augenkrankheit, wie Retina-Vaskularisierung, diabetische Retinopathie, altersbedingte Makula-Degeneration und dergleichen.
Gegenstand der Erfindung ist deshalb auch die Verwendung der erfindungsgemäßen Verbindungen gemäß Anspruch 1 zur Herstellung eines Medikaments zur Behandlung und/oder Prophylaxe der vorstehenden Erkrankungen.

Die Verwendung von erfindungsgemäßen Verbindungen gemäß Anspruch 1 und/oder ihrer physiologisch unbedenklichen Salze und Solvate zur Herstellung eines Medikaments zur Behandlung und/oder Prophylaxe von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.

Bevorzugt ist die Verwendung der Verbindungen gemäß Anspruch 1 zur Herstellung eines Medikaments zur Behandlung von Erkrankungen, vorzugsweise aus der Gruppe der hyperproliferativen und nichthyperproliferativen Erkrankungen.
Hierbei handelt es sich um Krebserkrankungen oder nicht-krebsartige Erkrankungen.

Gegenstand der Erfindung ist auch Verwendung erfindungsgemäßer Verbindungen gemäß Anspruch 1 und/oder ihrer physiologisch unbedenklichen Salze; Solvate und Stereoisomere, einschließlich deren Mischungen in allen Verhältnissen zur Herstellung eines Medikaments zur Behandlung von Krankheiten, ausgewählt aus der Gruppe der nicht-krebsartigen Erkrankungen bestehend aus Psoriasis, Arthritis, Entzündungen, Endometriose, Vernarbung, gutartiger Prostatahyperplasie, immunologischer Krankheiten, Autoimmunkrankheiten und Immunschwächekrankheiten.

Ein weiterer Gegenstand der Erfindung ist die Verwendung von erfindungsgemäßen Verbindungen gemäß Anspruch 1 und/oder ihrer physiologisch unbedenklichen Salze, Solvate und Stereoisomere, einschließlich deren Mischungen in allen Verhältnissen zur Herstellung eines Medikaments zur Behandlung von Krankheiten, ausgewählt aus der Gruppe der krebsartigen Erkrankungen bestehend aus Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischem Krebs, Schilddrüsenkrebs, Lymphom, multiplem Myelom, chronischer Leukämie und akuter Leukämie.

Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, zytotoxische Stoffe, antiproliferative Mittel, Prenyl-Proteintransferaseinhibitoren, HMG-CoA-Reduktase-Inhibitoren, HIV-Protease-Inhibitoren, Reverse-Transkriptase-Inhibitoren, Wachstumsfaktor-Inhibitoren sowie Angiogeneseinhibitoren. Die vorliegenden Verbindungen eignen sich insbesondere zur gemeinsamen Anwendung mit Radiotherapie.
"Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, wobei diese Aufzählung keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Inhibitoren, Nilutamid, Flutamid, Bicalutamid, Liarozol und Abirateronacetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenylretinamid.
"zytotoxische Stoffe" bezieht sich auf Verbindungen, die in erster Linie durch direkte Einwirkung auf die Zellfunktion zum Zelltod führen oder die die Zellmitose hemmen oder diese stören, darunter Alkylierungsmittel, Tumornekrosefaktoren, interkaliernde Mittel, Mikrotubulin-Inhibitoren und Topoisomerase-Inhibitoren.
Zu den zytotoxischen Stoffen zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diaminplatin(II)]bis[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyldaunorubicin (siehe WO 00/50032), was jedoch keine Einschränkung darstellen soll.

Zu den Mikrotubulin-Inhibitoren zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.

Topoisomerase-Inhibitoren sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]-pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNP11100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]acridin-6-on, N-[1-[2(Diethylamino)ethylamino]-7-methoxy-9-oxo-9H-thioxanthen-4-ylmethyl]formamid, N-(2-(Dimethylamino)-ethyl)acridin-4-carboxamid, 6-[[2-(Dimethylamino)-ethyl]amino]-3-hydroxy-7H-indeno[2,1-c]chinolin-7-on und Dimesna.

Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-mannoheptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehydthiosemicarbazon. Die "antiproliferativen Mittel" beinhalten auch monoklonale Antikörper gegen Wachstumsfaktoren wie Erbitux, Trastuzumab, sowie Tumorsuppressorgene, wie p53, die über rekombinanten virusvermittelten Gentransfer abgegeben werden können (siehe z. B. US-Patent Nr. 6,069,134).

### Ausführungsbeispiele

### Beispiel 1: Herstellung von (2-Chloro-pyrimidin-4-yl)-quinolin-3-yl-amin

50 g (0,34 mol) 2,4-Dichlorpyrimidin und 50 g (0,35 mol) 3-Aminoquinolin werden zusammengegeben und in 400 mL 2-Propanol mit 100 mL Ethyldiisopropylamin für 50 h unter Rückfluß am Sieden gehalten. Das Reaktionsgemsich wird auf 3 L Eiswasser gegossen, der Niederschlag abgesaugt und mit Wasser gewaschen. Der Rückstand wird aus Aceton umkristallisiert und man erhält 60g hellgraue Kristalle (Fp: 140-143°C) (2-Chloropyrimidin-4-yl)-quinolin-3-yl-amin.

### Beispiel 2: Herstellung von (4-Amino-phenyl)-(2,3-dihydro-1H-indol-7-yl)-methanon

5 g (42 mmol) Indolin werden in 50 mL Toluol gelöst. In einem separaten Kolben werden 70 mL Toluol auf 5°C abgekühlt und bei dieser Temperatur unter Stickstoff mit 100 mL Bortrichlorid (10%ige Lösung in Xylol) tropfenweise versetzt. Anschließend wird zu dieser Lösung bei 5-10°C das Indolin zugetropft und nachfolgend 5,4 g (46 mmol) 4-Aminobenzonitril innerhalb von 30 min portionsweise zugegeben. Es wird 15 min bei 5-10° nachgerührt und dann bei der angegebenen Temperatur portionsweise 6,7 g (50 mmol) Aluminiumchlorid zugegeben. Man erhitzt 6 h unter Rückfluß. Zur Aufarbeitung wird das Reaktionsgemisch auf 70°C abgekühlt und 10 mL Wasser zugetropft, wobei die Temperatur leicht ansteigt und die Lösung trüb wird. Anschließend gibt man noch 60 mL 2N Salzsäure zu, wobei wieder eine klare Lösung entsteht und erwärmt für 12 h unter Rückfluß. Das Reaktionsgemisch wird auf Eiswasser gegossen, mit konz. NaOH auf pH=12 gebracht und mit Ethylacetat extrahiert. Die vereinigten organischen Phasen werden über Natriumsulfat getrocknet und eingeengt und der Rückstand über eine Säule mit Essigsäureethylester chromatographiert. Die vereinigten Produktfraktionen werden aus Petrolether umkristallisiert und man erhält 3,8 g gelbe Kristalle (4-Amino-phenyl)-(2,3-dihydro-1H-indol-7-yl)-methanon. (Schmp. 133-135°C)

Nach diesem Protokoll werden ebenso hergestellt:
- (4-Benzyloxy-phenyl)-(2,3-dihydro-1H-indol-7-yl)-methanon
- (2,3-Dihydro-1H-indol-7-yl)-(4-hydroxy-phenyl)-methanon
- (2,3-Dihydro-1H-indol-7-yl)-pyridin-4-yl-methanon
- (2,3-Dihydro-1H-indol-7-yl)-pyridin-3-yl-methanon

Die nach dem oben beschriebenen Verfahren hergestellten Ketone lassen sich bei RT unter Normaldruck unter Verwendung von dem Fachmann bekannten Katalysatoren wie z.B. Pd/C zu den entsprechenden Alkoholen reduzieren. Unter Verwendung von Platin(IV)oxid wird neben dem Keton auch das Pyridin reduziert. Alternativ kann unter Verwendung von Reduktionsmitteln wie z. B. Natriumborhydrid die Carbonylgruppe selektiv reduziert werden. Die nachfolgenden Akoholderivate der Formel I können so erhalten werden:
- (1H-Indol-7-yl)-piperidin-4-yl-methanol
- (1H-Indol-7-yl)-pyridin-4-yl-methanol
- (2,3-Dihydro-1H-indol-7-yl)-piperidin-4-yl-methanol
- (2,3-Dihydro-1H-indol-7-yl)-pyridin-4-yl-methanol,
wobei ggf, die Indole aus den Indolinen durch Oxidation hergestellt werden, z. B. mit CrO3.

### Beispiel 3: Herstellung von (2,3-Dihydro-1H-indol-7-yl)-{4-[4-(quinolin-3ylamino)-pyrimidin-2-ylamino]-phenyl}-methanon

200 mg (0.84 mmol) (4-Amino-phenyl)-(2,3-dihydro-1H-indol-7-yl)-methanon und 215 mg (0.84 mmol) (2-Chloro-pyrimidin-4-yl)-quinolin-3-yl-amin werden in 2 mL DMSO für 2 h auf 120°C erwärmt. Die organische Phase wird mit Essigsäureethylester und Wasser verrührt und nach Phasentrennung, Trocknung und Eindampfung über Kieselgel chromatographisch aufgereinigt. Man erhält 500 mg (2,3-Dihydro-1H-indol-7-yl)-{4-[4-(quinolin-3ylamino)-pyrimidin-2-ylamino]-phenyl}-methanon. (Schmp.: 235-238°C)

Auf diese Weise wird auch hergestellt:
- (2,3-Dihydro-1H-indol-7-yl)-{4-[2-(quinolin-3-ylamino)-pyrimidin-4-ylamino]-phenyl}-methanon.

### Beispiel 4: Hemmung von IGF-1 R (IC₅₀)

Kultivierte humane Tumorzellen, die den IGF1-Rezeptor (IGF1R) exprimieren (z.B. MCF-7 oder Calu-6), werden mit humanem IGF1, dem natürlichen Liganden des IGF1R stimuliert. Die Stimulation induziert eine Autophosphorylierung von Tyrosinresten in der cytoplasmatischen IGF1R-Domäne, welche Signaltransduktionskaskaden auslöst, die zur Apoptosehemmung und Proliferation der Zellen führen.
Die Menge an phosphoryliertem IGF1R wird durch einen rezeptorspezifischen Capture-ELISA oder einen analogen LUMINEX-Assay bestimmt. Der IGF1R aus Zelllysaten wird mittels eines spezifischen Antikörpers an eine 96-well ELISA-Platte bzw. LUMINEX-Beads gebunden ("Capturing"), und die Tyrosinphosphorylierung mit einem Biotin-markierten anti-Phosphotyrosin Antikörper und einem Streptavidin-Peroxidase-Konjugat durch ein Chemilumineszenz-Verfahren bzw. mittels eine Fluoreszenzmarkierten anti-Phosphotyrosin-Antikörpers detektiert.
Zur Bestimmung der Aktivität von Kinaseinhibitoren werden Zellen mit ansteigenden Konzentrationen dieser Verbindungen für 45 min vorbehandelt und anschließend für 5 min mit IGF1 stimuliert. Als interne Kontrolle wird die biologische Aktivität des Liganden IGF1 überprüft sowie eine Konzentrationsreihe eines IGF1 R-Referenzinhibitors vermessen.

Für (2,3-Dihydro-1H-indol-7-yl)-{4-[4-(quinolin-3ylamino)-pyrimidin-2-ylamino]-phenyl}-methanon wird nach dieser Vorschrift folgendes Ergebnis erhalten: Die Substanz hemmt die Kinase IGF-1 R zu 50%, wenn die Verbindung in einer Konzentration von 120 nM vorliegt.

Weitere Inhibitionskonstanten von erfindungsgemäßen Verbindungen sind in der Tabelle 1 aufgeführt.

Die nachfolgenden Beispiele betreffen pharmazeutische Zubereitungen:

### Beispiel 5a: Injektionsgläser

Eine Lösung von 100 g eines erfindungsgemäßen Wirkstoffes und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel 5b: Suppositorien

Man schmilzt ein Gemisch von 20 g eines erfindungsgemäßen Wirkstoffes mit 100 g Sojalecithin und 1400 g Kakaobutter, gießt in Formen und lässt erkalten. Jedes Suppositorium enthält 20 mg Wirkstoff.

### Beispiel 5c: Lösung

Man bereitet eine Lösung aus 1 g eines erfindungsgemäßen Wirkstoffes, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel 5d: Salbe

Man mischt 500 mg eines erfindungsgemäßen Wirkstoffes mit 99,5 g Vaseline unter aseptischen Bedingungen.

### Beispiel 5e: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpresst, derart, dass jede Tablette 10 mg Wirkstoff enthält.

### Beispiel 5f: Dragees

Analog Beispiel 5e werden Tabletten gepresst, die anschließend in üblicher Weise mit einem Überzug aus Saccharose, Kartoffelstärke, Talk, Tragant und Farbstoff überzogen werden.

### Beispiel 5g: Kapseln

2 kg Wirkstoff werden in üblicher Weise in Hartgelatine kapseln gefüllt, so dass jede Kapsel 20 mg des Wirkstoffs enthält.

### Beispiel 5h: Ampullen

Eine Lösung von 1 kg eines erfindungsgemäßen Wirkstoffes in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe bestehend aus
(2,3-Dihydro-1H-indol-7-yl)-{4-[4-(quinolin-3-ylamino)-pyrimidin-2-ylamino]-phenyl}-methanon,
[4-(2-Chloro-pyrimidin-4-ylamino)-phenyl]-(1H-indol-7-yl)-methanon,
[4-(2-Chloro-pyrimidin-4-ylamino)-phenyl]-(2,3-dihydro-1H-indol-7-yl)-methanon,
(2,3-Dihydro-1H-indol-7-yl)-(4-{4-[(pyridin-3-ylmethyl)-amino]-pyrimidin-2-ylamino}-phenyl)-methanon,
(1H-Indol-7-yl)-{4-[2-(3,4,5-trimethoxy-phenylamino)-pyrimidin-4-yiamino]-phenyl}-methanon,
(4-{2-[4-(1H-Indole-7-carbonyl)-phenylamino]-pyrimidin-4-ylamino}-phenyl)-(1H-indol-7-yl)-methanon,
(4-{2-[4-(3-Cyano-1H-indole-7-carbonyl)-phenylamino]-pyrimidin-4-ylamino}-phenyl)-(3-cyano-1H-indol-7-yl)-methanon,
3-{4-[4-(1H-Indole-7-carbonyl)-phenylamino]-pyrimidin-2-ylamino}-propionitril,
(4-{2-[(3-Hydroxy-cyclobutylmethyl)-amino]-pyrimidin-4-ylamino}-phenyl)-(1H-indol-7-yl)-methanon,
{4-[2-(1H-Imidazol-2-ylamino)-pyrimidin-4-ylamino]-phenyl}-(1H-indol-7-yl)-methanone,
(1H-Indol-7-yl)-{3-[4-(quinolin-3-ylamino)-pyrimidin-2-ylamino]-1H-pyrazol-4-yl}-methanon,
(1H-Indol-7-yl)-{4-[2-(5-phenyl-1H-pyrazol-3-ylamino)-pyrimidin-4-ylamino]-phenyl}-methanon,
1H-Indol-7-yl)-{4-[2-(5-methyl-1H-pyrazol-3-ylamino)-pyrimidin-4-ylamino]-phenyl}-methanon,
{4-[2-(5-Furan-2-yl-1H-pyrazol-3-ylamino)-pyrimidin-4-ylamino]-phenyl}-(1H-indol-7-yl)-methanon,
{4-[2-(5-tert-Butyl-1H-pyrazol-3-ylamino)-pyrimidin-4-ylamino]-phenyl}-(1H-indol-7-yl)-methanon,
(1H-Indol-7-yl)-{4-[2-(1H-pyrazol-3-ylamino)-pyrimidin-4-ylamino]-phenyl}-methanon,
(2,3-Dihydro-1H-indol-7-yl)-{4-[2-(quinolin-3-ylamino)-pyrimidin-4-ylamino]-phenyl}-methanon,
sowie ihre pharmazeutisch unbedenklichen Salze, Solvate und Stereoisomere, einschließlich deren Mischungen in allen Verhältnissen.

2. Verbindungen Anspruch 1 und/oder ihre physiologisch unbedenklichen Salze, Solvate und Stereoisomere, einschließlich deren Mischungen in allen Verhältnissen als Arzneimittel.

3. Arzneimittel, enthaltend wenigstens eine Verbindung nach Anspruch 1 und/oder ihre physiologisch unbedenklichen Salze, Solvate und Stereoisomere, einschließlich deren Mischungen in allen Verhältnissen, sowie gegebenenfalls Träger- und/oder Hilfsstoffe.

4. Arzneimittel, enthaltend wenigstens eine Verbindung nach einem oder mehreren der Ansprüche 1 bis 8 und/oder ihre physiologisch unbedenklichen Salze, Solvate und Stereoisomere, einschließlich deren Mischungen in allen Verhältnissen sowie wenigstens einen weiteren Arzneimittelwirkstoff.

5. Set (Kit) bestehend aus getrennten Packungen von
a) einer wirksamen Menge einer Verbindung nach Anspruche 1 und/oder ihrer physiologisch unbedenklichen Salze, Solvate und Stereoisomere, einschließlich deren Mischungen in allen Verhältnissen und
b) einer wirksamen Menge eines weiteren Arzneimittelwirkstoffs.

6. Verbindungen nach einem Anspruch 1 sowie ihre physiologisch unbedenklichen Salze, Solvate und Stereoisomere, einschließlich deren Mischungen in allen Verhältnissen als Aktivatoren oder Inhibitoren von Kinasen, insbesondere Tyrosin-Kinasen.

7. Verbindungen nach Anspruch 1 sowie ihre physiologisch unbedenklichen Salze, Solvate und Stereoisomere, einschließlich deren Mischungen in allen Verhältnissen als Inhibitoren der Rezeptor-Tyrosinkinase IGF-1 R.

8. Verwendung von Verbindungen nach Anspruch 1 und/oder ihrer physiologisch unbedenklichen Salze, Solvate und Stereoisomere, einschließlich deren Mischungen in allen Verhältnissen zur Herstellung eines Medikaments zur Prophylaxe oder Behandlung von Krankheiten, bei denen die Hemmung der Rezeptor-Tyrosinkinase IGF-1R zur Verbesserung des Krankheitsbildes führt.

9. Verwendung von Verbindungen nach einem Anspruch 1 und/oder ihrer physiologisch unbedenklichen Salze, Solvate und Stereoisomere, einschließlich deren Mischungen in allen Verhältnissen zur Herstellung eines Medikaments zur Prophylaxe oder Behandlung von Krebs, Tumorwachstum, Tumorangiogenese, Arteriosklerose, der diabetischen Retinopathie und Entzündungserkrankungen.

10. Verwendung von Verbindungen nach Anspruch 1 und/oder ihrer physiologisch unbedenklichen Salze, Solvate und Stereoisomere, einschließlich deren Mischungen in allen Verhältnissen zur Herstellung eines Medikaments zur Prophylaxe oder Behandlung von Brustkrebs, Prostatakrebs, Kolorektalkrebs, kleinzelligem Lungenkrebs, nicht-kleinzelligem Lungenkrebs, multiplem Myelom sowie dem Nierenzellkarzinom und dem Endometriumkarzinom.

## Claims

1. Compounds selected from the group consisting of
(2,3-dihydro-1H-indol-7-yl)-{4-[4-(quinolin-3-ylamino)pyrimidin-2-ylamino]phenyl}methanone,
[4-(2-chloropyrimidin-4-ylamino)phenyl]-(1H-indol-7-yl)methanone,
[4-(2-chloropyrimidin-4-ylamino)phenyl]-(2,3-dihydro-1H-indol-7-yl)-methanone,
(2,3-dihydro-1H-indol-7-yl)-(4-{4-[(pyridin-3-ylmethyl)amino]pyrimidin-2-ylamino}phenyl)methanone,
(1H-indol-7-yl)-{4-[2-(3,4,5-trimethoxyphenylamino)pyrimidin-4-ylamino]phenyl}methanone,
(4-{2-[4-(1H-indole-7-carbonyl)phenylamino]pyrimidin-4-ylamino}-phenyl)-(1H-indol-7-yl)methanone,
(4-{2-[4-(3-cyano-1H-indole-7-carbonyl)phenylamino]pyrimidin-4-ylamino}phenyl)-(3-cyano-1H-indol-7-yl)methanone,
3-{4-[4-(1H-indole-7-carbonyl)phenylamino]pyrimidin-2-ylamino}-propionitrile,
(4-{2-[(3-hydroxycyclobutylmethyl)amino]pyrimidin-4-ylamino}-phenyl)-(1H-indol-7-yl)methanone,
{4-[2-(1H-imidazol-2-ylamino)pyrimidin-4-ylamino]phenyl}-(1H-indol-7-yl)methanone,
(1H-indol-7-yl)-{3-[4-(quinolin-3-ylamino)pyrimidin-2-ylamino]-1H-pyrazol-4-yl}methanone,
(1H-indol-7-yl)-{4-[2-(5-phenyl-1H-pyrazol-3-ylamino)pyrimidin-4-ylamino]phenyl}methanone,
(1H-indol-7-yl)-{4-[2-(5-methyl-1H-pyrazol-3-ylamino)pyrimidin-4-ylamino]phenyl}methanone,
{4-[2-(5-furan-2-yl-1H-pyrazol-3-ylamino)pyrimidin-4-ylamino]-phenyl}-(1H-indol-7-yl)methanone,
{4-[2-(5-tert-butyl-1H-pyrazol-3-ylamino)pyrimidin-4-ylamino]-phenyl}-(1H-indol-7-yl)methanone,
(1H-indol-7-yl)-{4-[2-(1H-pyrazol-3-ylamino)pyrimidin-4-ylamino]-phenyl}methanone,
(2,3-dihydro-1H-indol-7-yl)-{4-[2-(quinolin-3-ylamino)pyrimidin-4-ylamino]phenyl}methanone,
and pharmaceutically acceptable salts, solvates and stereoisomers thereof, including mixtures thereof in all ratios.

2. Compounds according to Claim 1 and/or physiologically acceptable salts, solvates and stereoisomers thereof, including mixtures thereof in all ratios, as medicaments.

3. Medicaments comprising at least one compound according to Claim 1 and/or physiologically acceptable salts, solvates and stereoisomers thereof, including mixtures thereof in all ratios, and optionally excipients and/or adjuvants.

4. Medicaments comprising at least one compound according to Claim 1 and/or physiologically acceptable salts, solvates and stereoisomers thereof, including mixtures thereof in all ratios, and at least one further medicament active ingredient.

5. Set (kit) consisting of separate packs of
a) an effective amount of a compound according to Claim 1 and/or physiologically acceptable salts, solvates and stereoisomers thereof, including mixtures thereof in all ratios, and
b) an effective amount of a further medicament active ingredient.

6. Compounds according to Claim 1 and physiologically acceptable salts, solvates and stereoisomers thereof, including mixtures thereof in all ratios, as activators or inhibitors of kinases, in particular tyrosine kinases.

7. Compounds according to Claim 1 and physiologically acceptable salts, solvates and stereoisomers thereof, including mixtures thereof in all ratios, as inhibitors of the receptor tyrosine kinase IGF-1R.

8. Use of compounds according to Claim 1 and/or physiologically acceptable salts, solvates and stereoisomers thereof, including mixtures thereof in all ratios, for the preparation of a medicament for the prophylaxis or treatment of diseases in which inhibition of the receptor tyrosine kinase IGF-1R results in an improvement in the clinical picture.

9. Use of compounds according to Claim 1 and/or physiologically acceptable salts, solvates and stereoisomers thereof, including mixtures thereof in all ratios, for the preparation of a medicament for the prophylaxis or treatment of cancer, tumour growth, tumour angiogenesis, arteriosclerosis, diabetic retinopathy and inflammatory diseases.

10. Use of compounds according to Claim 1 and/or physiologically acceptable salts, solvates and stereoisomers thereof, including mixtures thereof in all ratios, for the preparation of a medicament for the prophylaxis or treatment of breast cancer, prostate cancer, colorectal cancer, small-cell lung cancer, non-small-cell lung cancer, multiple myeloma and renal cell carcinoma and endometrial carcinoma.

## Revendications

1. Composés choisis dans le groupe constitué par
la (2,3-dihydro-1H-indol-7-yl)-{4-[4-(quinoléin-3-ylamino)pyrimidin-2-ylamino]phényl}méthanone,
la [4-(2-chloropyrimidin-4-ylamino)phényl]-(1H-indol-7-yl)méthanone,
la [4-(2-chloropyrimidin-4-ylamino)phényl]-(2,3-dihydro-1H-indol-7-yl)méthanone,
la (2,3-dihydro-1H-indol-7-yl)-(4-{4-[(pyridin-3-ylméthyl)amino]-pyrimidin-2-ylamino}phényl)méthanone,
la (1H-indol-7-yl)-{4-[2-(3,4,5-triméthoxyphénylamino)pyrimidin-4-ylamino]phényl}méthanone,
la (4-{2-[4-(1H-indole-7-carbonyl)phénylamino]pyrimidin-4-ylamino}phényl)-(1H-indol-7-yl)méthanone,
la (4-{2-[4-(3-cyano-1H-indole-7-carbonyl)phénylamino]pyrimidin-4-ylamino}phényl)-(3-cyano-1H-indol-7-yl)méthanone,
le 3-{4-[4-(1H-indole-7-carbonyl)phénylamino]pyrimidin-2-ylamino}propionitrile,
la (4-{2-[(3-hydroxycyclobutylméthyl)amino]pyrimidin-4-ylamino}-phényl)-(1H-indol-7-yl)méthanone,
la {4-[2-(1H-imidazol-2-ylamino)pyrimidin-4-ylamino]phényl}-(1H-indol-7-yl)méthanone,
la (1H-indol-7-yl)-{3-[4-(quinoléin-3-ylamino)pyrimidin-2-ylamino]-1H-pyrazol-4-yl}méthanone,
la (1H-indol-7-yl)-{4-[2-(5-phényl-1H-pyrazol-3-ylamino)pyrimidin-4-ylamino]phényl}méthanone,
la (1H-indol-7-yl)-{4-[2-(5-méthyl-1H-pyrazol-3-ylamino)pyrimidin-4-ylamino]phényl}méthanone,
la {4-[2-(5-furan-2-yl-1H-pyrazol-3-ylamino)pyrimidin-4-ylamino]-phényl}-(1H-indol-7-yl)méthanone,
la {4-[2-(5-tertio-butyl-1H-pyrazol-3-ylamino)pyrimidin-4-ylamino]-phényl}-(1H-indol-7-yl)méthanone,
la (1H-indol-7-yl)-{4-[2-(1H-pyrazol-3-ylamino)pyrimidin-4-ylamino]-phényl}méthanone,
la (2,3-dihydro-1H-indol-7-yl)-{4-[2-(quinoléin-3-ylamino)pyrimidin-4-ylamino]phényl}méthanone,
et des sels, solvats et stéréoisomères pharmaceutiquement acceptables de ceux-ci, y compris des mélanges de ceux-ci en toutes proportions.

2. Composés selon la revendication 1 et/ou des sels, solvats et stéréoisomères physiologiquement acceptables de ceux-ci, y compris des mélanges de ceux-ci en toutes proportions, comme médicaments.

3. Médicaments comprenant au moins un composé selon la revendication 1 et/ou des sels, solvats et stéréoisomères physiologiquement acceptables de celui-ci, y compris des mélanges de ceux-ci en toutes proportions, et éventuellement des excipients et/ou des adjuvants.

4. Médicaments comprenant au moins un composé selon la revendication 1 et/ou des sels, solvats et stéréoisomères physiologiquement acceptables de celui-ci, y compris des mélanges de ceux-ci en toutes proportions, et au moins un autre ingrédient actif médicamenteux.

5. Ensemble (kit) constitué de conditionnement séparés
a) d'une quantité efficace d'un composé selon la revendication 1 et/ou de sels, solvats et stéréoisomères physiologiquement acceptables de celui-ci, y compris des mélanges de ceux-ci en toutes proportions, et
b) d'une quantité efficace d'un autre ingrédient actif médicamenteux.

6. Composés selon la revendication 1 et sels, solvats et stéréoisomères physiologiquement acceptables de ceux-ci, y compris des mélanges de ceux-ci en toutes proportions, comme activateurs ou inhibiteurs de kinases, en particulier de tyrosine kinases.

7. Composés selon la revendication 1 et sels, solvats et stéréoisomères physiologiquement acceptables de ceux-ci, y compris des mélanges de ceux-ci en toutes proportions, comme inhibiteurs du récepteur à activité de tyrosine kinase IGF-1 R.

8. Utilisation de composés selon la revendication 1 et/ou de sels, solvats et stéréoisomères physiologiquement acceptables de ceux-ci, y compris des mélanges de ceux-ci en toutes proportions, pour la préparation d'un médicament destiné à la prophylaxie ou au traitement de maladies dans lesquelles une inhibition du récepteur à activité de tyrosine kinase IGF-1 R entraîne une amélioration du tableau clinique.

9. Utilisation de composés selon la revendication 1 et/ou de sels, solvats et stéréoisomères physiologiquement acceptables de ceux-ci, y compris des mélanges de ceux-ci en toutes proportions, pour la préparation d'un médicament destiné à la prophylaxie ou au traitement du cancer, de la croissance tumorale, de l'angiogenèse tumorale, de l'artériosclérose, de la rétinopathie diabétique et des maladies inflammatoires.

10. Utilisation de composés selon la revendication 1 et/ou de sels, solvats et stéréoisomères physiologiquement acceptables de ceux-ci, y compris des mélanges de ceux-ci en toutes proportions, pour la préparation d'un médicament destiné à la prophylaxie ou au traitement du cancer du sein, du cancer de la prostate, du cancer colorectal, du cancer du poumon à petites cellules, du cancer du poumon non à petites cellules, du myélome multiple et du carcinome à cellules rénales et du carcinome de l'endomètre.
